# EUROPEAN PATENT APPLICATION

(11) **EP 4 403 181 A1**
(43) Date of publication of application: **24.07.2024**
(21) Application number: 23180396.6
(22) Date of filing: 20.06.2023
(51) Int. Cl.: A61K 38/07, A61P 25/04, A61P 29/00

(54) **A MEDICINAL PRODUCT FOR USE IN ADMINISTERING IN THE TREATMENT, CONTROL AND/OR PREVENTION OF PAIN**

(30) Priority: 18.01.2023 RU 2023101017
(71) Applicant: PVP Labs PTE. Ltd., Singapore 307684 (SG)
(72) Inventor: Kosorukov, Vyacheslav Stanislavovich, 142152 Podolsk (RU); Abuzarova, Guzal Rafailovna, Moscow (RU); Gamzeleva, Olesya Yuryevna, Moscow (RU)
(74) Representative: Spengler, Robert

(57) **Abstract**

The proposed group of inventions pertains to medicine, and specifically, to a medicinal product for use in administering in the treatment, control and prevention of pain using the tetrapeptide Tyrosyl-D-arginyl-phenylalanyl-glycinamide (H-Tyr-D-Arg-Phe-Gly-NH₂) and especially to a medicinal product on the basis of this tetrapeptide which can be used for acute or chronic pain, in particular, in cancer patients and in the early postoperative period after various surgical interventions.

## Description

The proposed group of inventions pertains to medicine, and specifically, to a medicinal product for use in administering in the treatment, control and prevention of pain using the tetrapeptide Tyrosyl-D-arginyl-phenylalanyl-glycinamide (H-Tyr-D-Arg-Phe-Gly-NH₂) and especially to a medicinal product on the basis of this tetrapeptide which can be used for acute or chronic pain, in particular, in cancer patients and in the early postoperative period after various surgical interventions.

Acute and chronic pain is one of the most intolerable and psychologically taxing manifestations of disease, including cancer, and of the early postoperative period after a variety of surgical interventions. High prevalence of pain necessitates continuous search for and selection of new treatment regimens for its prevention or alleviation.

The present-day spectrum of pharmacological analgesics includes various groups of drugs: opioid analgesics, non-steroidal anti-inflammatory drugs (NSAIDs), combination products, corticosteroids, anticonvulsants, etc. However, the search for new analgesics and therapy methods based on their application remains an important problem, as known analgesics have a number of serious drawbacks. Opioid drugs are known to be associated with a high risk of drug dependence in chronic pain patients and a have wide range of adverse events (AEs), including nausea, drowsiness, cognitive decline, fatigue, elevated risk of balance impairment (risk of falling), immunosuppression, etc. Unlike opioid drugs, other products, such as NSAIDs, corticosteroids and anticonvulsants, are not associated with a high risk of drug dependence or AEs, but their analgesic effectiveness is not always sufficient, especially in cancer patients.

Development of peptide-based analgesia methods is an area of particular focus in the search for new methods of pain treatment, prevention and control. In particular, a number of publications described high analgesic activity of the tetrapeptides H-Tyr-D-Arg-Phe-Gly-NH₂ and H-Tyr-D-Arg-Phe-Sar-OH (JPS58213743 (A), published 12.12.1983, and JPS6054400 (A), published 28.03.1985).

A method for the prevention and/or treatment of acute or chronic pain comprising administration of a medication on the basis of the tetrapeptide H-Tyr-D-Arg-Phe-Gly-NH₂ or H-Tyr-D-Arg-Phe-Sar-OH in the quantity of 0.5 - 10 mg/dose (patent RU 2622980) was selected as the prototype of the proposed invention.

The prototype method specifies a wide range of single doses: from 0.5 to 10 mg/dose; according to the complete description of the patent, this range was determined "based on the data of extrapolation... for humans" of results obtained in a study of effects of the tetrapeptide H-Tyr-D-Arg-Phe-Gly-NH₂ or H-Tyr-D-Arg-Phe-Sar-OH "on the behavioral reactions of animals", in large-dose toxicity studies in the open field test and in the test for maintaining equilibrium on a horizontal rod (Example 8 of patent RU 2622980). However, no large-scale clinical trials were performed. Example 9 of patent RU 2622980 contains details on the administration of these tetrapeptides to six patients only; additionally, "the prototype of the drug was used in cases where the patient was shown treatment with morphine group painkillers (stage 3 according to the WHO system), but for one reason or another the drug was not available. When a regular morphine drug appeared, therapy with an experimental drug prototype was discontinued". Out of the six patients, five had stage IV cancer of different body locations (pancreatic cancer; colon cancer; breast cancer), and one had vertebral fracture. A product on the basis of the tetrapeptide H-Tyr-D-Arg-Phe-Gly-NH₂ or the tetrapeptide H-Tyr-D-Arg-Phe-Sar-OH was administered by subcutaneous injection at doses of 0.7 to 3 mg twice daily (usually for 4-5 days, when the clinic for some reason was out of morphine, which was prescribed for analgesia). The analgesic effect, according to the description in patent RU 2622980, manifested in pain reduction over 10-15 minutes after dosing, with the analgesic effect persisting for 4-10 hours.

As the therapeutic range of the tetrapeptide in prototype method was selected "by extrapolation... for humans" of results obtained by studying the tetrapeptide's effects "on the behavioral reactions of animals", and no large-scale clinical trials were performed, the safety and efficacy of the proposed range of single therapeutic doses (0.5-10 mg/dose) remains questionable. Adequate justifications for the efficacy of the minimum dose of the specified range (0.5 mg), safety of the maximum dose (10 mg) and for the specific doses selected for clinical use in the six patients (0.7 mg, 1 mg, 1.5 mg, 3 mg) are lacking. The prototype method also lacks details on the maximum daily dose of the tetrapeptide. Neither is there information on the maximum duration of use of the prototype method for analgesia (as mentioned earlier, clinical trials in the prototype method include details on the use of the drug in only six patients and only in the short periods during which morphine, which had been prescribed to these patients, was for whatever reason unavailable). Additionally, specific examples of the use of the drug in patients failed to specify which of the two tetrapeptides proposed in the prototype method (the tetrapeptide H-Tyr-D-Arg-Phe-Gly-NH₂ or the tetrapeptide H-Tyr-D-Arg-Phe-Sar-OH) was used in each individual patient; therefore, one cannot be confident that clinical efficacy was confirmed specifically for the tetrapeptide H-Tyr-D-Arg-Phe-Gly-NH₂.

As a consequence, there exists a pressing need to improve the efficacy and safety of analgesia with the tetrapeptide H-Tyr-D-Arg-Phe-Gly-NH₂ by developing optimum doses, regimens and courses of treatment with this tetrapeptide on a more solid foundation, based on large-scale clinical trials.

It is also important to develop a medicinal product on the basis of the tetrapeptide H-Tyr-D-Arg-Phe-Gly-NH₂ for using it as a medicinal product in the treatment and/or prevention and/or control of pain.

Patent RU 2622980, which was selected as a prototype, describes a means for the treatment and/or prevention of pain on the basis of the tetrapeptide H-Tyr-D-Arg-Phe-Gly-NH₂ or H-Tyr-D-Arg-Phe-Sar-OH, and in particular, an injectable product prepared in the dosage form of a solution. Claims 12 and 15 describe liquid dosage form compositions, which comprise specific excipients at specific ratios.

Claim 12 of the prototype describes a pharmaceutical composition that includes the tetrapeptide H-Tyr-D-Arg-Phe-Gly-NH₂ or H-Tyr-D-Arg-Phe-Sar-OH and excipients, such as buffer, filler, stabilizer and solvent, and specifies a range of possible content values for each component. However, these content ranges of the components remained in part hypothetical, as they were not corroborated by specific examples in the complete description of the patent. Specifically, the claims specified the range from 0.01 to 0.5 wt % for the tetrapeptide, whereas embodiments only list compositions with a tetrapeptide content of 0.15 wt %. Additionally, the patent description lacks information on compositions with tetrapeptide content in mg other than 1.5 mg (the tetrapeptide content in all the liquid injectable compositions listed in the embodiments was 1.5 mg). Therefore, the development of a drug for use in the treatment, control and/or prevention of pain remains to represent a pressing need.

It is an object of the present invention to improve the efficacy and safety of the medicinal product for use in the treatment and/or prevention and/or control of pain, in particular, in cancer patients, as well as during the early postoperative period after a variety of surgical interventions by developing optimum doses, regimens and courses of treatment with the tetrapeptide H-Tyr-D-Arg-Phe-Gly-NH₂.

The object is solved by a medicinal product for use in administering in the treatment, control and/or prevention of pain according to any one of claims 1, 12 or 20. Advantageous modifications and developments are detailed in the subclaims.

The formulation "an administration pattern includes administering the tetrapeptide Tyrosyl-D-arginyl-phenylalanyl-glycinamide (H-Tyr-D-Arg-Phe-Gly-NH₂)" means that the tetrapeptide Tyrosyl-D-arginyl-phenylalanyl-glycinamide (H-Tyr-D-Arg-Phe-Gly-NH₂) is to be administered to a patient in need according to the administration pattern.

In an aspect of the invention, the object is solved by a medicinal product for use in the treatment of pain, wherein an administration pattern of the medicinal product includes administering the tetrapeptide Tyrosyl-D-arginyl-phenylalanyl-glycinamide (H-Tyr-D-Arg-Phe-Gly-NH₂) at a single dose of 2 mg to 7 mg, for a daily dose not exceeding 42 mg, with a dosing interval of 4 hours or more. A duration of the pain treatment course is preferably up to 96 days, with the possibility of repeating the course multiple times.

This medicinal product has the following individual aspects:
Preferably, the medicinal product is a medical product for use in administering to a human in the treatment, control and/or prevention of pain. According to a preferred embodiment the administration pattern includes an administration for pain relief in cancer patients and in the early postoperative period after a variety of surgical interventions.

Preferably, the administration pattern includes administering the tetrapeptide Tyrosyl-D-arginyl-phenylalanyl-glycinamide (H-Tyr-D-Arg-Phe-Gly-NH₂) in the form of a solution for subcutaneous injection.

In a preferred embodiment the administration pattern includes administering the tetrapeptide while monitoring the severity of pain and/or the patient's response to the tetrapeptide, and increasing or decreasing as necessary the single dose and/or the number of doses per day and/or the dosing interval and/or the duration of the course of treatment. I.e., the single dose and/or the number of doses per day and/or the dosing interval and/or the duration of the course of treatment are increased or decreased as necessary depending on the monitoring of the severity of pain and/or the patient's response to the tetrapeptide.

Preferably, the administration pattern includes that, depending on pain severity, tetrapeptide injections are performed daily or with pauses of one to several days.

In a preferred embodiment, the administration pattern includes determining the total duration of pain treatment by the patient's condition. In cancer patients, the duration of a single course of pain treatment may be up to 96 days or more, with the possibility of repeating the course multiple times; repeating the course is recommended after a pause of at least 2-5 days between the courses, with the number of repeat courses being determined by the patient's condition.

Preferably, the administration pattern includes determining the total duration of treatment by the patient's condition. In patients with pain in the early postoperative period after a variety of surgical interventions, the duration of pain treatment is preferably 1 to 10 days.

In a preferred embodiment the application pattern includes using a single 4 mg to 7 mg dose of the tetrapeptide.

In a preferred embodiment the administration pattern includes a recommended starting single dose of the tetrapeptide Tyrosyl-D-arginyl-phenylalanyl-glycinamide (H-Tyr-D-Arg-Phe-Gly-NH₂) which is 4 mg. It was demonstrated by the inventors that optimum compositions for the production of medicinal product on the basis of the tetrapeptide H-Tyr-D-Arg-Phe-Gly-NH₂ comprise the tetrapeptide at levels much greater than 0.15 wt % (or 1.5 mg) as described by RU 2622980, since, as will be shown below, a recommended starting dose of the tetrapeptide of 4 mg is advantageous.

Preferably, the administration pattern includes increasing or reducing as necessary the single dose in line with an increase or reduction of the dose by 25-50% relative to the previous value.

In a preferred embodiment the application pattern includes administering the tetrapeptide at a dose of 4 mg 2-3 times daily. It was demonstrated by the inventors that optimum compositions for the production of medicinal product on the basis of the tetrapeptide H-Tyr-D-Arg-Phe-Gly-NH₂ comprise the tetrapeptide at levels much greater than 0.15 wt % (or 1.5 mg) as disclosed by RU 2622980, since, as will be shown below, an optimum course for pain treatment, control and/or prevention including administering 4 mg of the tetrapeptide 2-3 times daily is advantageous.

This object is also achieved by proposing a medicinal product for use in the control of pain, wherein the administration pattern includes administering the tetrapeptide Tyrosyl-D-arginyl-phenylalanyl-glycinamide (H-Tyr-D-Arg-Phe-Gly-NH₂) at a single dose of 2 mg to 7 mg, for a daily dose not exceeding 42 mg, and with a dosing interval of 4 hours or more. Individual aspects of this medicinal product are as outlined above in relation to the treatment of pain or in the subclaims, respectively.

This object is also achieved by proposing a medicinal product for use in the prevention of an attack of pain, wherein the administration pattern includes administering the tetrapeptide Tyrosyl-D-arginyl-phenylalanyl-glycinamide (H-Tyr-D-Arg-Phe-Gly-NH₂) at a single dose of 2 mg to 7 mg, for a daily dose not exceeding 42 mg, and with a dosing interval of 4 hours or more. Individual aspects of this medicinal product are as outlined above in relation to the treatment of pain or in the subclaims, respectively

In a preferred embodiment, the medicinal product comprises the tetrapeptide Tyrosyl-D-arginyl-phenylalanyl-glycinamide (H-Tyr-D-Arg-Phe-Gly-NH₂) and excipients: sodium chloride, mannitol, glycine, sodium acetate trihydrate, acetic acid and water at the following component ratios, wt %:

| | |
|---|---|
| Tetrapeptide | 0.380 - 0.420 |
| Sodium chloride | 0.475 - 0.525 |
| Mannitol | 0.475 - 0.525 |
| Glycine | 0.475 - 0.525 |
| Sodium acetate trihydrate | 0.0380 - 0.0420 |
| Acetic acid | up to pH 4.0 - 6.0 |
| Water | Up to 100. |

The proposed medicinal product is characterised by the following preferred embodiments:
Preferably, the medicinal product is characterized by the following ratio of the components, wt %:

| | |
|---|---|
| Tetrapeptide | 0.40 |
| Sodium chloride | 0.50 |
| Mannitol | 0.50 |
| Glycine | 0.50 |
| Sodium acetate trihydrate | 0.04 |
| Acetic acid | up to pH 4.0 - 6.0 |
| Water | up to 100. |

Preferably, the medicinal product is characterized by the following ratio of the components, mg:

| | |
|---|---|
| Tetrapeptide | 3.8 - 4.2 |
| Sodium chloride | 4.75 - 5.25 |
| Mannitol | 4.75 - 5.25 |
| Glycine | 4.75 - 5.25 |
| Sodium acetate trihydrate | 0.38 - 0.42 |
| Acetic acid | up to pH 4.0 - 6.0 |
| Water | up to 1 mL |

Preferably, the medicinal product is characterized by the following ratio of the components, mg:

| | |
|---|---|
| Tetrapeptide | 4.00 |
| Sodium chloride | 5.00 |
| Mannitol | 5.00 |
| Glycine | 5.00 |
| Sodium acetate trihydrate | 0.40 |
| Acetic acid | up to pH 4.0 - 6.0 |
| Water | up to 1 mL. |

Preferably, the medicinal product is or is prepared in the dosage form of a solution for subcutaneous injection.

Unless specified otherwise, all the technical and scientific terms used in this document have meanings that are commonly received among specialists in the fields pertinent to the invention. For any terms that have multiple definitions, the definition described in this application shall apply. It should be pointed out that if a URL or a similar address or identifier is cited as a reference, the specific identifier can obviously change, and specific information in the Internet can appear or disappear, but equivalent information can be found online using search engines. A reference to a specific piece of information is meant to indicate that this information is available and can be disseminated. The preceding brief description and the following detailed description of the invention are provided for reference only and shall in no way restrict any of the claimed subjects. The use of the terms "which comprise", "including" and their synonyms shall not be construed as confining or limiting in any way. It should be noted that the methods and compositions described in this invention are not limited to the specific methodologies, protocols, constructs and reagents that are described in this document and may vary. It should also be mentioned that the terminology used in this document is used solely for the purpose of describing specific embodiments of the invention and shall not confine the scope of the described methods and compositions, which are confined only by the claims of the invention attached.

Clinical trials have been performed, which involved administering the tetrapeptide Tyrosyl-D-arginyl-phenylalanyl-glycinamide (H-Tyr-D-Arg-Phe-Gly-NH₂) to patients by subcutaneous injection of the finished medicinal product Taphalgin.

The tetrapeptide Tyrosyl-D-arginyl-phenylalanyl-glycinamide (H-Tyr-D-Arg-Phe-Gly-NH₂) is a highly specific µ1-opioid receptor agonist, which exhibits almost no interaction with the other opioid receptors (interaction with δ-receptors is 3 orders of magnitude weaker than with µ-opioid receptors, and no interactions with the other receptors have been detected). Two subtypes of µ receptors are recognized: activation of µ1 receptors causes pronounced analgesia, while activation of µ2-receptors leads to respiratory depression, cardiovascular system disturbances such as bradycardia, and suppression of intestinal motility. Therefore, high specificity of the tetrapeptide in question to µ1-opioid receptors ensures the absence of side effects that are typical of most opioid narcotic medications. This tetrapeptide interacts with peripheral and central receptors, although it mostly acts at the spinal level. After the tetrapeptide binds to µ1-receptors, G-protein complex is released, which inhibits the release of neurotransmitters by the cell by reducing the amount of cAMP produced, closing calcium channels and opening potassium channels, which, among other consequences, inhibits the transmission of pain signals in nociceptors. The tetrapeptide activates the antinociceptive system, thereby disrupting interneuronal transmission of pain signals at different levels of the CNS, and changes the intensity of pain perception by acting on the higher brain centres.

Taphalgin is a solution for subcutaneous injection, which includes the tetrapeptide Tyrosyl-D-arginyl-phenylalanyl-glycinamide (H-Tyr-D-Arg-Phe-Gly-NH₂) as the active ingredient, as well as excipients including sodium acetate trihydrate, sodium chloride, mannitol, glycine and water at the following component ratios:
the tetrapeptide Tyrosyl-D-arginyl-phenylalanyl-glycinamide: 0.4 wt %, sodium acetate trihydrate: 0.04 wt %, sodium chloride: 0.5 wt %, mannitol: 0.5 wt %, glycine:
0.5 wt %, water: up to 100 wt %.

The content of the ingredients may vary within 15-20 % of their respective values specified above.

Taphalgin 4 mg/mL was used.

For the preparation of Taphalgin, excipients are dissolved in water, then the peptide is dissolved in the resulting solution, the solution is sterilized, filtered and filled in ampoules or syringes.

An initial phase I clinical trial was performed on 39 healthy male volunteers in order to evaluate the safety, tolerability and pharmacokinetics of Taphalgin after subcutaneous administration. This study was carried out in cohorts with dose escalation for each subsequent cohort (0.05 mg; 0.5 mg; 1 mg; 2 mg; 3 mg; 4 mg; 5 mg; 6 mg; and 7 mg). Taphalgin was administered once by subcutaneous injection starting from the lowest-dose cohort. Each participant received one dose only. Dose escalation for each subsequent cohort was only performed after a comprehensive evaluation of AEs / serious AEs (SAEs), vital signs, electrocardiography results, physical examination results and laboratory tests, as well as data on the incidence of effects of dose-limiting toxicity (DLT). If DLTs were identified in one person who received the investigational product in any cohort, the number of volunteers would have to be increased by three. If none of the volunteers of the expanded cohort taking Taphalgin developed dose-limiting toxicity (i.e. if DLTs were observed in 1 out of 6 subjects), dose escalation in the next cohort could continue. In order to evaluate the pharmacokinetics of Taphalgin, 22 blood samples were collected from each volunteer (before dosing (0 h), at 10, 20, 30 and 45 min and 1; 1,5;2; 2,5; 3; 4; 5; 6; 7; 8; 10; 12; 24; 48; 72; 96 and 120 h after dosing). The following parameters were calculated: area under the curve within the follow-up period (AUC₀₋ₜ), area under the curve from dosing to infinity (AUC_{0-∞}), maximum concentration (*C*ₘₐₓ), time to maximum concentration (*T*ₘₐₓ), half-life (*T*_{½}), mean residence time (MRT) in blood plasma, elimination rate constant (*K*ₑₗ) and volume of distribution (*V*_{d}). Taphalgin concentration in blood plasma was determined by high-performance liquid chromatography with tandem mass spectrometry (HPLC-MS/MS) using a Triple Quad 4500 system (AB Sciex) [49]. At the final stage of the study, clinical and biochemical blood tests were also performed, and coagulation parameters were investigated. The total study duration for each volunteer did not exceed 14 days. The study demonstrated good safety and tolerability of different doses of Taphalgin (0.05 mg; 0.5 mg; 1 mg; 2 mg; 3 mg; 4 mg; 5 mg; 6 mg; and 7 mg) when administered to healthy volunteers once. The highest dose that was achieved by dose escalation was 7 mg, and no cases of dose-limited toxicity were recorded (AE severity grade 3 or more according to the Common Terminology Criteria for Adverse Events, CTCAE). In the investigator's evaluation, the tolerability of Taphalgin was described as "good" or "excellent" in all patients. The most common AE reported during the study (over 5 % of the total number of AEs) were: asthenia *(n* = 11; 28.20 %), xerostomia *(n* = 11; 28.20 %), hypaesthesia (*n =* 9; 23.08 %) and discomfort in the head (*n =* 9; 23.08). The observed AEs were mild, did not require treatment, and almost all resolved with complete recovery.

The time to maximum concentration of the drug after subcutaneous injection was, on average, 30 minutes, indicating rapid absorption from subcutaneous fat tissue. After a single administration, Taphalgin is actively distributed not just in the bloodstream, but in the peripheral tissues as well, as indicated by the volume of distribution (24 to 39 L on average), which far exceeds the volume of circulating blood. Distribution was followed by a rather rapid elimination stage: the half-life of the drug was 1.3 to 2.5 h; therefore, it was completely eliminated from systemic circulation in approximately 15 hours.

In general, similar elimination figures are typical of therapeutic polypeptides, of which Taphalgin is a representative. The mean residence time of one molecule of the drug in the human body was 3.03 ± 0.42 h.

A phase II clinical trial evaluated the efficacy and safety of various doses of Taphalgin in cancer patients who were experiencing pain. Additionally, the optimum dosing regimen was selected, and its pharmacokinetic parameters with multiple dosing were evaluated. Pain intensity was evaluated on a numeric rating scale (NRS) from 0 to 10. The study was performed on patients of both sexes, 18 to 70 years of age inclusively, with a documented diagnosis of cancer and severe cancer pain that was controlled by morphine at the time of enrolment (NRS pain intensity score at screening < 3, maximum daily dose of morphine 60 mg IM, receiving a stable dose or morphine for at least 7 days before screening) and a life expectancy of over 1 month. The patients did not receive analgesics other than those specified in the protocol. The patients' physical activity score on the Eastern Cooperative Oncology Group (ECOG) performance status scale was 0 to 3, i.e. they were active and were up and about for over 50 % of waking hours (grade 2 on the ECOG scale) or were capable of only limited self-care and confined to bed or chair more than 50% of waking hours (grade 3 on the ECOG scale). The study was carried out in two stages. The total duration of the trial for each patient was 20 days or less. At stage 1 (duration: 10 days), the dose of Taphalgin was selected: each participant received the initial single dose of the drug to which they were randomized (2, 3, 4, 5, 6 or 7 mg) followed by titration according to the established procedure. The number of doses depended on pain intensity as evaluated on the NRS scale for each individual patient. A parallel-group design with different starting doses in each group was chosen based on the fact that the phase I clinical trial demonstrated acceptable safety profile of that various doses of Taphalgin that were investigated in that trial. At stage 2, patients received either Taphalgin (solution for subcutaneous injection at a personalized dose selected at stage 1; maximum single dose = 7 mg, maximum daily dose = 42 mg) or the comparator drug morphine (solution for intramuscular injection at a personalized dose selected before the start of stage 1).

In the event of breakthrough pain, morphine was to be used in the form of solution for injections 10 mg / 1 mL.

The comparator drug was selected due to its widespread use in this type of patients and based on results of preclinical trials of Taphalgin, which justified a hypothesis that it was comparable to morphine in the strength of its analgesic effect. The primary efficacy endpoint was the rate of achieving a sufficient analgesic effect throughout the day. This effect was understood as mandatory compliance with two criteria, viz. achieving a daily mean NRS pain intensity score < 3 and the requiring not more than 2 morphine injections (10 mg IM) for controlling breakthrough pain within 10 days. The following secondary endpoints were additionally selected to confirm the analgesic efficacy of Taphalgin:
- trends in daily average NRS pain intensity score (in points);
- hourly mean NRS pain intensity score between the first and second dose at the 10th visit;
- trends in the daily dose of the additional analgesic for controlling breakthrough pain (in mg);
- nocturnal sleep quality on the Likert 3-point scale based on the patient diary (the duration of nocturnal sleep uninterrupted by pain was scored as follows: 1 = good sleep quality (6-8 hours), 2 = fair quality (4-6 hours), 3 = poor quality (less than 4 hours);
- frequency of drug dose increases;
- prevalence of the need for changing treatment.

The patients were split into 6 groups that received different initial doses of Taphalgin:
group 1 *(n* = 6): 2 mg; group 2 *(n* = 6): 3 mg; group 3 *(n* = 12): 4 mg; group 4 *(n* = 6): 5 mg; group 5 *(n* = 6): 6 mg; group 6 *(n* = 6): 7 mg. All the groups had comparable demographics (*p* > 0.05).

At stage 1, analysis revealed statistically significant differences in daily average NRS pain intensity scores between study days in groups that received Taphalgin at doses of 2 mg *(p* <0.00001), 3 mg *(p* <0.03681), 4 mg *(p* <0.00131), 6 mg *(p* <0.00497) and 7 mg *(p* <0.01788), indicating favourable trends in pain severity in these groups and a reduction of pain intensity associated with the study drug. A comparative analysis of daily average NRS pain intensity scores on days 1-10 revealed no significant differences between the study groups, suggesting that while the average within-subgroup daily dose on day 10 was 8.33 mg, even the lowest investigated single dose of Taphalgin (2 mg) exhibited sufficient efficacy, whereas the average starting dose of morphine in this subgroup was 18.33 mg.

At stage 2 of the study, no significant differences in NRS pain intensity scores between study days were found in the morphine group; therefore, the within-group trend in pain sensations was flat (*p* = 0.88079). The trend of daily average NRS pain intensity scores in the Taphalgin group also remained flat, i.e. the patients' pain sensations were stable (*p* = 0.46607). Therefore, the product controlled pain intensity as well as morphine, as evidenced by between-group comparison indicating that the groups did not differ by pain intensity throughout stage 2 (*p* > 0.05).

**Table 1**

| Trends in daily average Numeric Rating Scale (NRS) pain intensity scores at stage 2 of the trial. | | | |
|---|---|---|---|
| Day of dosing | Taphalgin | Morphine | p |
| 11 | 0.07 ± 0.16 | 0.12 ± 0.22 | 0.4392 |
| 12 | 0.18 ± 0.34 | 0.30 ± 0.38 | 0.5581 |
| 13 | 0.19 ± 0.26 | 0.24 ±0.35 | 0.6814 |
| 14 | 0.16 ± 0.25 | 0.24 ± 0.35 | 0.5061 |
| 15 | 0.09 ± 0.22 | 0.28 ± 0.30 | 0.1252 |
| 16 | 0.14 ± 0.19 | 0.17±0.21 | 0.3721 |
| 17 | 0.11 ± 0.15 | 0.17 ± 0.23 | 0.4656 |

Quality of sleep analysis demonstrated an improvement in patients who were treated with Taphalgin: by study days 9-10, sleep quality was fair in 76-79 % and good in 20-23 % of patients.

Before the start of the study, all the patients participating in stage 1 of the trial were taking morphine at a personalized dose that ensured adequate pain relief. At baseline, the average stable daily dose of this product for all the Taphalgin dose subgroups was 20.31 mg; 14 (33.33 %) patients were receiving morphine at a dose of 10 mg; 18 (42.86 %), at a dose of 20 mg; 6 (14.29 %), at a daily dose of 30 mg; 3 (7.14 %), at a daily dose of 40 mg; and 1 (2.38 %), at a dose of 60 mg. Taphalgin dose was adjusted at stage 1 until a daily dose ensuring adequate pain relief was achieved. Average daily dose of the drug at Visit 10 was 8.25 mg in pooled subgroups, 8.33 mg in dose subgroup 2, 6.80 mg in dose subgroup 3, 10.10 mg in dose subgroup 4, 7.25 mg in dose subgroup 5, 8.80 mg in dose subgroup 6, and 6.00 mg in dose subgroup 7. As a result, individualized daily dozes of morphine and Taphalgin could be compared for each patient evaluated in the study. The patients were receiving a stable dose of the drug by, on average, day 6 or 7 at stage 1 of the study. The daily average dose for days 7 through 10 was included in the analysis and compared to the pre-determined morphine dose for each patient. In general, the daily average dose of Taphalgin was 3 times less than the morphine dose for the same patient.

Findings for adverse events (AEs) are presented in Table 2 below.

**Table 2**

| Number of adverse events (AEs) reported during stage 2 of the study, N (% of the total number of AEs for the corresponding group) | | |
|---|---|---|
| AE | Morphine | Taphalgin |
| Anaemia | 1 (3.7) | 0 (0) |
| Asthenia | 4 (14.8) | 0 (0) |
| Insomnia | 1 (3.7) | 0 (0) |
| Dizziness | 1 (3.7) | 0 (0) |
| Dizziness postural | 0 (0) | 1 (33.3) |
| Retention of urine | 1 (3.7) | 0 (0) |
| Constipation | 4 (14.8) | 2 (66.7) |
| Cough | 1 (3.7) | 0 (0) |
| Shortness of breath | 1 (3.7) | 0 (0) |
| Peripheral oedema | 1 (3.7) | 0 (0) |
| Vomiting | 1 (3.7) | 0 (0) |
| Sedation | 2 (7.4) | 0 (0) |
| Appetite lost | 2 (7.4) | 0 (0) |
| Dry mouth | 1 (3.7) | 0 (0) |
| Nausea | 1 (3.7) | 0 (0) |
| Flatulence | 1 (3.7) | 0 (0) |
| Acute heart failure (SAE) | 1 (3.7) | 0 (0) |
| Pulmonary oedema (SAE) | 1 (3.7) | 0 (0) |
| Multiple organ dysfunction syndrome (SAE) | 1 (3.7) | 0 (0) |
| Total | 27 (100) | 3 (100) |

### Comments: 1. There were a total of 32 patients in both groups, 16 in each. 2. SAE = serious adverse event.

At stage 2 of the study, 30 SAEs were reported in 10 (31.25 %) of 32 patients. AE incidence analysis in the Taphalgin and morphine groups at stage 2 of the study is presented in Table 2. Safety profile was shown to be significantly more favourable in the Taphalgin group than in the morphine group: the incidence of AEs in the morphine group was 50.0%, with 27 AEs reported in 8 patients and 1 SAE reported in 1 patient of the morphine group. The incidence of AEs in the Taphalgin group was just 12.5 %, with 3 AEs being reported in 2 patients of this group. A comparative analysis of AEs revealed a significant difference between the groups (*p* = 0.022). At the same time, there were no significant between-group differences in AE severity, causal association between AEs and therapy or AE outcomes.

Additionally, trends in opioid-associated conditions were evaluated in patients who were switched from morphine to Taphalgin; the incidence of opioid-associated conditions was shown to decrease throughout the 10 days of dose titration: whereas their incidence was 59.52 % (25 / 42) at baseline, it dropped to 14.29 % (6 / 42) at the end of the 10 days of Taphalgin treatment. Therefore, far from causing opioid-associated AEs, Taphalgin contributes to reducing these AEs in patients who had been receiving morphine.

These data indicate considerable clinical efficacy of Taphalgin. The use of this product allowed to maintain adequate analgesia, which had been achieved by intramuscular injections of morphine, throughout the study. Data on dosing frequency demonstrate that even in the low dosage range (2-4 mg), which has a favourable safety profile, the drug can control tumour-associated pain for 8-12 hours. However, in order to achieve adequate pain control, the dose of Taphalgin must be selected on a case-by-case basis, as the pathogenesis of pain, intensity of pain perception and pain severity are subject to individual variation. Whereas the clinically effective dose is 2 mg, the other doses up to 7 mg are well tolerated and are not associated with dose-limiting toxicities. The maximum daily dose of Taphalgin must not exceed 42 mg. The drug must be administered with a dosing interval of not less than 4 hours.

None of the patients receiving Taphalgin exhibited sedation associated with reduced alertness and concentration. Drowsiness, memory and concentration deficits are classical AEs that develop with opioid use and substantially impact patients' quality of life and work capacity.

Another important factor is that Taphalgin, unlike opioid drugs, does not inhibit the respiratory centre: none of the patients receiving this product exhibited dyspnoea, whereas difficulty breathing was observed in the morphine group.

Therefore, these investigations showed that the tetrapeptide Tyrosyl-D-arginyl-phenylalanyl-glycinamide (H-Tyr-D-Arg-Phe-Gly-NH₂) should be administered for pain at a single dose of 2-7 mg and daily dose of not more than 42 mg, with a dosing interval of 4 hours or more.

The recommended initial dose is 4 mg. The dose and multiplicity of dosing are then individualized taking into account pain severity and response to treatment. If the single dose must be increased, this should be done in steps of 25-50% of the previous dose level. If the dose level must be reduced (if a higher dose produces a good therapeutic effect or in the event of AEs), this should also be done in steps of 25-50% of the previous dose.

These recommendations were then used in the next series of clinical trials (phase III clinical trials).

Further clinical trials were performed in 180 male and female patients 18 to 80 years of age with cancer pain. The patients were split in two groups:
- group 1 patients (n=120) received Taphalgin, a solution for subcutaneous administration in an individually selected dose throughout the study.
- group 2 patients (n=59) were receiving an oral preparation of morphine as a comparator drug for 28 days from the end of the dose selection period (Period 1). This was followed (Period 2) by reassigning the patients of this group to Taphalgin solution for subcutaneous injection, which was administered at an individualized dose until the end of participation in the clinical trial.

For patients who were randomized to receive the study drug, the initial dose of Taphalgin solution for subcutaneous injection was 4 mg. The single dose of the drug was increased or decreased (if necessary) in 2 mg steps. The maximum single dose of Taphalgin was 7 mg. The maximum daily dose of Taphalgin was 42 mg. The duration of treatment with the study drug did not exceed 94±2 days.

Primary efficacy evaluation was performed were during the following visits: Visit 1 (day 1 of tetrapeptide treatment), Visit 2 (day 28±2 of tetrapeptide treatment), Visit 3 (day 56±2 of tetrapeptide treatment) and Visit 4 (day 86±2 of tetrapeptide treatment).

### Efficacy endpoints:

The following endpoints were used for treatment efficacy evaluation:
Primary endpoint:
   Analgesic treatment response maintenance rate by day 28.
   Treatment response was considered maintained if all of the following conditions were met:
      1. Not more than 2 dose increases of the test drug / comparator drug from the start of therapy at the selected dose to day 28 of therapy were required.
      2. Daily average pain intensity at the end of the 28-day treatment period remains at <50% of the intensity at the start of titration.
Secondary endpoints:
   - Proportion of patients who required a dose increase by Visits 2, 3 and 4 from the start of therapy at the selected dose.
   - Proportion of patients who failed to respond to Taphalgin/morphine treatment by Visits 2, 3 and 4.
   - Proportion of patients with at least one episode of breakthrough pain by Visits 2, 3 and 4 from the start of therapy at the selected dose.
   - Trends in daily average NRS pain intensity score by Visits 2, 3 and 4 from the start of therapy at the selected dose.
   - Proportion of patients who required concomitant analgesic therapy by Visits 2, 3 and 4.
   - Patients' quality of life scores based on the SF-36 questionnaire.
   - Therapy efficacy as evaluated by the Investigator by the end of the dose selection period at Visit 1 and by Visit 2.
   - Therapy efficacy as evaluated by the patient by the end of the dose selection period at Visit 1 and by Visit 2.
Safety endpoints:
   - Total number of severity- and frequency-stratified adverse events (AEs);
   - Proportion of adverse drug reactions;
   - Proportion of serious adverse events (SAEs) associated with Taphalgin/morphine treatment;
   - Proportion of patients with at least one reported AE;
   - Proportion of patients who discontinued treatment due to AEs.

The proportion of patients with analgesic therapy response retention by day 28 was 98.97 % (96/97) in Group 1 (Taphalgin) and 100 % (46/46) in Group 2 (morphine). The difference in the proportion of patients with analgesic treatment response retention by day 28 between Group 1 (Taphalgin) and Group 2 (morphine) was 0.0103 (1.03 %). The lower bound of the 95 % CI for the proportion difference was -0.0862 (8.62 %), and the upper bound, 0.0643 (6.43 %).

A comparative analysis of the proportion of patients with analgesic therapy response retention by day 28 failed to identify statistically significant differences between Group 1 (Taphalgin) and Group 2 (morphine) (single-sided Fisher's exact test, p=0.6783).

The proportion of patients who required a dose increase by Visit 2 was 4.12 % (4/97) in Group 1 (Taphalgin) and 6.52 % (3/46) in Group 2 (morphine). A comparative analysis of the proportion of patients who required a dose increase by Visit 2 failed to identify statistically significant differences between Group 1 (Taphalgin) and Group 2 (morphine) (Fisher's exact test, p=0.6809).

By Visit 2, there were no non-responders to either Taphalgin or morphine therapy in Group 1 (Taphalgin) or 2 (morphine). A comparative analysis of the proportion of non-responders to Taphalgin/morphine therapy by Visit 2 failed to identify statistically significant differences between Group 1 (Taphalgin) and Group 2 (morphine) (Pearson's chi-square test, p=1.0000).

No non-responders to Taphalgin therapy were identified by Visit 3.

No non-responders to Taphalgin therapy were identified by Visit 4.

The proportion of patients with at least one episode of breakthrough pain by Visit 2 was 0.00 % (0/97) in Group 1 (Taphalgin) and 2.17 % (1/46) in Group 2 (morphine). A comparative analysis of the proportion of patients with at least one episode of breakthrough pain by Visit 2 failed to identify statistically significant differences between Group 1 (Taphalgin) and Group 2 (morphine) (Fisher's exact test, *p*=0.3217)*.*

The proportion of patients with at least one episode of breakthrough pain by Visits 3 and 4 despite treatment with Taphalgin was 1.40 % (2/143).

Daily average NRS pain intensity score for the entire population (Mean±SD) was 0.38±0.47 at the start of therapy at the selected dose and 0.34±0.40 at Visit 2. Daily average NRS pain intensity score in Group 1 (Taphalgin) (Mean±SD) was 0.42±0.48 at the start of therapy at the selected dose and 0.38±0.44 at Visit 2. Daily average NRS pain intensity score in Group 2 (morphine) (Mean±SD) was 0.30±0.47 at the start of therapy at the selected dose and 0.28±0.30 at Visit 2.

Daily average NRS pain intensity score for the entire population (Mean±SD) was 0.38±0.46 at the start of therapy at the selected dose, 0.35±0.54 at Visit 3 and 0.35±0.65 at Visit 4. Analysis failed to identify significant differences between the visits (p=0.0770), suggesting a flat trend in daily average NRS pain intensity scores by Visit 3 and 4 with Taphalgin treatment.

The average SF-36 physical health score in the entire study population was 33.91±11.39 at Visit 0 and 51.18±11.50 at Visit 2. The average SF-36 physical health score in Group 1 (Taphalgin) was 34.71±11.64 at Visit 0 and 51.96±11.36 at Visit 2. The average SF-36 physical health score in Group 2 (morphine) was 32.22±10.76 at Visit 0 and 49.52±11.73 at Visit 2.

The average SF-36 mental health score in the entire study population was 34.84±13.12 at Visit 0 and 48.13±10.22 at Visit 2. The average SF-36 mental health score in Group 1 (Taphalgin) was 36.46±13.30 at Visit 0 and 49.06±9.71 at Visit 2. The average SF-36 mental health score in Group 2 (morphine) was 31.42±12.16 at Visit 0 and 31.42±12.16 at Visit 2.

At Visit 2 in Group 1 (Taphalgin), the proportion of patients with an "excellent response" to therapy was 55.67 % (54/97), with a "good response", 41.24 % (40/97), and with a "satisfactory response", 3.09 % (3/97). At Visit 2 in Group 2 (morphine), the proportion of patients with an "excellent response" to therapy was 32.61 % (15/46), with a "good response", 67.39 % (31/46), and with a "satisfactory response", 0.00 % (0/46). A comparative analysis of the proportion of patients with various investigator-evaluated therapy efficacy scores at Visit 2 revealed statistically significant differences between the study groups (Pearson's chi-square test, p=0.0103).

The study included the following safety evaluation stages: Dose selection; Period 1, the comparative stage of the study (28±2 days from the start of therapy at the selected dose); and Period 2, the non-comparative stage of the study (56±2 days after Period 1).

The prevalence of patients with AEs reported in Group 1 (Taphalgin) during Period 1 was 34.17 % (41/120): a total of 95 AEs were reported in 41 patients. The prevalence of patients with AEs reported in Group 2 (morphine) during Period 1 was 71.19 % (42/59): a total of 119 AEs were reported in 42 patients. A comparative analysis of Group 1 and 2 patients for AEs in Period 1 revealed significant differences between the study groups (Pearson's chi-square test, *p*<0.00001).

The AEs reported during Period 1 in Group 1 patients were mild in 66.32 % (63/95) of cases and moderate in 33.68 % (32/95) of cases; in Group 2 patients, mild in 65.55 % (78/119) of cases, moderate in 33.61 % (40/119) of cases and severe in 0.84 % (1/119) of cases. A comparative analysis revealed no significant differences between Groups 1 and 2 in terms of AE severity (p=0.6693).

Based on Investigator assessments, the causality between the AEs and therapy in Period 1 in Group 1 was considered certain in 1.05 % (1/95) of cases, probable in 3.16 % (3/95) of cases, possible in 44.21 % (42/95), unlikely in 13.68 % (13/95) of cases, conditional in 12.63 % (12/95) of cases, unclassifiable in 1.05 % (1/95) of cases, unrelated in 24.21 % (23/95) of cases; in Group 2, it was considered certain in 13.44 % (16/119) of cases, probable in 29.41 % (35/119) of cases, possible in 38.66 % (46/119) of cases, unlikely in 11.76 % (14/119) of cases, conditional in 0.84 % (1/119) of cases, unclassifiable in 0.84 % (1/119) of cases, and unrelated in 5.04 % (6/119) of cases. A comparative analysis of causality between AEs and treatment in Period 1 in Groups 1 and 2 revealed significant differences between the study groups (Pearson's chi-square test, *p<0.00001).*

In order to further evaluate the differences between Groups 1 and 2, the AEs were comprehensively analysed within the groups. This evaluation demonstrated that the prevalence of patients with reported AEs was lower in Group 1 (Taphalgin) at 36.69 % (44/120) vs. 72.88 % (43/59) in Group 2 (morphine). Additionally, the number of cases in which the Investigator reported causality between AEs and treatment as "certain", "probable" or "possible" was higher in Group 2 (morphine): there were 46 AEs in Group 1 (Taphalgin) vs. 97 AEs in Group 2 (morphine); additionally, in Period 1, there were no withdrawals from treatment due to AEs in Group 1, which received Taphalgin.

The prevalence of patients with AEs and/or SAEs reported during Period 2 was 12.74 % (20/157). A total of 35 AEs and/or SAEs were reported in 20 patients during Period 2. The prevalence of patients with AEs reported during Period 2 was 7.64 % (12/157). A total of 24 AEs were reported in 12 patients during Period 2. The AEs reported during Period 2 were mild in 54.17 % (13/24) of cases and moderate in 45.83 % (11/24) of cases. Based on Investigator assessments, the causality between the AEs reported in Period 2 and therapy with the study drug Taphalgin was considered certain in 16.67 % (4/24) of cases, possible in 33.33 % (8/24) of cases, unlikely in 12.50 % (3/24) of cases, conditional in 4.17 % (1/24) of cases and unrelated in 33.33 % (8/244) of cases. Analysis of AE outcome frequencies demonstrated that by the end of Period 2, recovery without sequelae was observed in 83.33 % (20/24) of cases, and improvement was observed in 16.67 % (4/24) of cases.

In general, safety evaluation demonstrated that the prevalence of patients with reported adverse events was lower in the Taphalgin group at 36.69 % (44/120) vs. 72.88 % (43/59) in the morphine group. Additionally, the number of cases in which the Investigator reported causality between adverse events and treatments as "certain", "probable" or "possible" was higher in the morphine group: there were 46 adverse events in the Taphalgin group vs. 97 adverse events in the morphine group.

Additional clinical trials focused of the treatment of pain in the early postoperative period after a variety of surgical interventions, including urological, gynaecological and other surgeries. The studies were performed in 100 patients who were split in 5 groups of 20 people each; three groups received Taphalgin at various doses (2 mg 2 times daily, 4 mg 2 times daily and 4 mg 3 times daily), and two groups received the comparator drug Promedol (20 mg 3 times daily).

Treatment with Taphalgin (or the comparator drug Promedol) started on the first day after surgery if NRS pain intensity score was ≥ 4. If NRS pain intensity score was ≥ 4 on day 2, Taphalgin was administered at the same dose and at the same dosing intervals, but if necessary, the dosing regimen could be adjusted within the day (the number of doses was usually reduced due to establishing pain control). If NRS pain intensity score on day two was ≤ 4, only paracetamol was administered by IV injection as necessary, and the maximum daily dose was 4 mg. The total duration of treatment did not exceed 8 days. Pain was usually brought under control within 1-2 days in the early postoperative period.

The proportion of patients who achieved greater than 50 % pain relief at 1 hour after receiving the initial dose was 65.0 % in the group receiving treatment with Taphalgin 4 mg twice daily, 55.0 % in the group receiving Taphalgin 2 mg three times daily, 80.0 % in the group receiving Taphalgin 4 mg three times daily and 50 % in the group receiving Promedol 20 mg three times daily.

Daily average NRS pain intensity score was 2.35 ± 0.70 points in the group receiving treatment with Taphalgin 2 mg three times daily, 2.51 ± 0.84 in the group receiving Taphalgin 4 mg twice daily, 2.14 ± 0.94 in the group receiving Taphalgin 4 mg three times daily and 2.16 ± 0.66 in the group receiving Promedol 20 mg three times daily. On day 2, this score was 1.45 ± 2.06 in the group receiving Taphalgin 2 mg three times daily, 1.48 ± 1.83 in the group receiving Taphalgin 4 mg twice daily, 0.92 ± 1.77 in the group receiving Taphalgin 4 mg three times daily and 1.41 ± 1.92 in the group receiving Promedol 20 mg three times daily. The mean change in pain intensity was 60.0 ± 25.45 % in the group receiving Taphalgin 2 mg three times daily, 57.56 ± 21.12 % in the group receiving Taphalgin 4 mg twice daily, 67.21 ± 20.11 % in the group receiving Taphalgin 4 mg three times daily, and 55.44 ± 33.17 % in the group receiving Promedol 20 mg three times daily.

Among the patients who required repeat dosing on day 2 in the group receiving Taphalgin 2 mg 3 times daily on day 1, 16.67 % were dosed three times, 66.67 % were dosed once, and 16.67 % were dosed twice.

Among the patients who required repeat dosing on day 2 in the group receiving Taphalgin 4 mg twice daily on day 1, 50.00 % were dosed twice, and 50.00 % were dosed once.

Among the patients who required repeat dosing on day 2 in the group receiving Taphalgin 4 mg three times daily on day 1, 11.11 % were dosed three times, and 89.89 % were dosed twice.

Among the patients who required repeat dosing on day 2 in the group receiving Promedol 20 mg three times daily on day 1, 16.67 % were dosed three times, and 83.33 % were dosed twice.

AEs were observed in 23 patients out of 100. The AEs were mild in 76.74 % of patients and moderate in 23.53 %. AEs were observed in 35 % of patients in the groups treated with Promedol, in 15 % of patients treated with Taphalgin 2 mg three times daily, in 10 % of patients treated with Taphalgin 4 mg twice daily, and in 20 % of patients treated with Taphalgin 4 mg three times daily. Based on Investigator assessments, the causality between the AEs and therapy with the study drug or comparator drug was considered certain in 73.53 %, probable in 5.88 %, possible in 11.76 %, unlikely in 5.88 %, and no relation was established in 2.94 % of cases.

The proposed medicinal product for use in administering to a human is embodied as follows:
Control and/or treatment and/or prevention of pain is carried out using a product on the basis of the tetrapeptide Tyrosyl-D-arginyl-phenylalanyl-glycinamide (H-Tyr-D-Arg-Phe-Gly-NH₂). In a particular embodiment, it can be Taphalgin, which is a solution for subcutaneous injection, which includes the tetrapeptide Tyrosyl-D-arginyl-phenylalanyl-glycinamide (H-Tyr-D-Arg-Phe-Gly-NH₂) (4 mg/mL) as the active ingredient, as well as excipients including sodium acetate trihydrate, sodium chloride, mannitol, glycine and water at the following component ratios:
the tetrapeptide Tyrosyl-D-arginyl-phenylalanyl-glycinamide: 0.4 wt %, sodium acetate trihydrate: 0.04 wt %, sodium chloride: 0.04 wt %, mannitol: 0.04 wt %, glycine: 0.04 wt %, water: up to 100 wt %.

The content of the ingredients may vary within 15-20 % of their respective values specified above.

The product is to be administered to the patient at a single dose of 2-7 mg 1-6 times daily at the maximum daily dose of 42 mg and dosing intervals of 4 hours or more. The recommended initial dose is 4 mg. The specific dose is selected on a case-by-case basis depending on pain severity, analgesic effect achieved after dosing, and presence or absence of AEs (adverse events) after dosing. Dose selection is usually performed in the first days of treatment, whereupon the severity of pain is monitored during treatment and, if necessary, the dose and number of daily doses are adjusted. The recommended initial dose of the product, as mentioned above, is 4 mg. The recommended optimum dosing regimen is 4 mg 2-3 times daily. If the single dose must be increased, this should be done in steps of 25-50% of the previous dose. If the dose must be reduced, this should also be done in steps of 25-50% of the previous dose.

Taphalgin can be used for long-term therapy; the duration of treatment is usually determined by the attending physician.

The course of treatment in phase III clinical trials in cancer patients lasted for up to 96 days. In phase III clinical trials in patients with early postoperative pain after a variety of surgical interventions the treatment usually lasted for 1 to 10 days.

If the product is used to treat chronic pain, it should preferably be dosed at regular intervals according to a fixed regimen.

In the event of long-term use, the need for extending treatment with the drug should be verified at regular intervals (such as by making short pauses in treatment), and the dose should be reviewed. For this reason, treatment courses of up to 96 days may be repeated multiple times, but pauses of at least 2-5 days should be made between the courses.

The tetrapeptide Tyrosyl-D-arginyl-phenylalanyl-glycinamide (H-Tyr-D-Arg-Phe-Gly-NH₂) may be used for preventing attacks of pain against a background of chronic pain. This is especially important for cancer patients. In such cases, the product is administered on a regular basis according to a fixed regimen, but doses are administered for preventing attacks of pain and not just during episodes of intense pain.

The proposed medicinal product for use in administering in the treatment, control and/or prevention of pain and administration patterns thereof are illustrated by the following examples.

### Example 1.

Patient N, age 65 years, male.

Diagnosis: sarcoma of the left retroperitoneum, T2NIMI St.IV; metastases in the peritoneum and bone. Chronic severe pain.

The patient is undergoing treatment on an outpatient basis, he is capable of self-care but cannot perform work. He spends over 50% of waking time up and about (50-60 points on the Karnofsky scale).

Before administering a treatment course according to the proposed administration pattern, pain was managed with morphine at a dose of 20 mg per day. This was accompanied by opioid-associated adverse events (AEs) such as dizziness, sleep disturbances, sedation, weakness and constipation.

Pain was treated according to the proposed administration pattern using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 3 mg twice daily at 12-hour intervals (at 9 am and 9 pm) for 14 days. Pain intensity was evaluated 0.5-1.5 hours before treatment, immediately before treatment, at 1 hour after treatment and at 1 pm daily. NRS pain score was usually estimated at 1-2 (in isolated cases, 3 or 0) before treatment and always declined to 0 after treatment (pain intensity score was usually 0 at one hour after dosing; it was usually 0-1 at the 1 pm evaluation). The patient remarked improved physical and mental status and sleep improvements.

Reported AEs were restricted to two transient episodes of mild dizziness and two episodes of constipation. However, AE relatedness to treatment with Taphalgin was not estimated as definite. The AEs were self-limiting.

### Example 2.

Patient N, age 64 years, male.

Diagnosis: cancer of the middle third of the oesophagus, T4NOMI St IV. Chronic severe pain.

The patient is undergoing treatment on an outpatient basis, he is capable of self-care but cannot perform work. He spends over 50% of waking time up and about (50-60 points on the Karnofsky scale).

Before administering a treatment course according to the proposed administration pattern, pain was managed with morphine at a dose of 20 mg per day. This was accompanied by opioid-associated adverse events (AEs) such as sedation and constipation.

The proposed administration pattern of pain control was employed using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 4-6 mg twice daily at 12-hour intervals (at 9 am and 9 pm) for 14 days. Pain intensity was evaluated 0.5-1.5 hours before treatment, immediately before treatment, at 1 hour after treatment and at 1 pm daily. During the first 1.5 days, the drug was administered at a single dose of 6 mg, and subsequently, at 4 mg. NRS pain intensity score was estimated at 3 on day 1 before treatment, 3 on day 2 at 9 am and 2 at 9 pm. Pain intensity score declined to 0-1 after dosing. On days 3-14, the product was administered at a dose of 4 mg twice daily. Pain intensity score was usually 1 (rarely 2) before dosing and declined to 0 after dosing. At the 1 pm evaluation, pain intensity was also reported as 0 (rarely 1). The patient remarked improved physical and mental status and sleep improvements.

AEs were observed as isolated episodes of mild insomnia and xerostomia. However, AE relatedness to treatment with Taphalgin was not estimated as definite. The AEs were self-limiting.

### Example 3.

Patient N, age 68 years, male.

Diagnosis: malignant neoplasm of the left retromolar space, T3NIMI 3 st. Keratinizing squamous cell carcinoma. Chronic pain (pain intensity score 2-3).

The patient cannot perform hard work but can perform light or sedentary work (such as household chores or office work) (70-80 on the Karnofsky scale).

Before administering a course according to the proposed administration pattern, morphine was used at a dose of 30 mg per day for controlling pain. This was accompanied by opioid-associated adverse events (AEs) such as sedation.

The proposed administration pattern for pain control was employed using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 2-4 mg two or three times daily at intervals of 6 or 12 hours for 14 days. Pain intensity was evaluated 0.5-1.5 hours before treatment, immediately before treatment, at 1 hour after treatment and at 1 pm daily. During the first four days, the product was administered at a dose of 4 mg twice daily. Before treatment with the drug, NRS pain intensity score was usually 2-4 (in isolated cases, 5 or 1) and always declined to 0-1 after dosing. On days 5-6 the product was administered at a dose of 2 mg twice daily. Pain intensity was usually given a score of 1 before dosing and 0 after dosing. On day 7, the product was administered in two doses of 2 mg each and a third dose of 4 mg; the dose was increased due to a pain intensity score of 5. Post-dosing pain intensity score was 2. On days 8-14, the product was administered at a dose of 4 mg three times daily at intervals of 6-8 hours. Pain intensity score declined from 2-4 to 0-2.

The patient remarked improved physical and mental status and sleep improvements.

AEs were observed as several episodes of mild drowsiness, loss of appetite and mild fever. However, AE relatedness to treatment with Taphalgin was estimated as unlikely.

### Example 4.

Patient N, age 64 years, male.

Diagnosis: cancer of unknown primary location, metastases in the subclavicular lymph node, liver, retroperitoneal lymph nodes, inguinal lymph nodes and bones. Chronic pain. The patient is undergoing treatment on an outpatient basis, he is capable of self-care but cannot perform work. He spends over 50% of waking time up and about (50-60 points on the Karnofsky scale).

Before administering a course according to the proposed administration pattern, morphine was used at a dose of 30 mg per day for controlling pain.

Pain was treated according to the proposed administration pattern using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 4-5 mg three times daily at intervals of 6 or 8 hours for 14 days. Pain intensity was evaluated 0.5-1.5 hours before treatment, immediately before treatment, at 1 hour after treatment and at 1 pm daily. During the first 2 days, the drug was administered at a single dose of 4 mg, and subsequently, at 5 mg. NRS pain intensity score was estimated at 2-4 on day 1 before treatment and 3-4 on day 2. Pain intensity score declined to 2 after dosing. On days 3-14, the product was administered at a dose of 5 mg three times daily. Pain intensity score was usually equal to 1 before dosing and declined to 0 after dosing. The patient remarked improved physical and mental status and sleep improvements.

AEs were observed as isolated episodes of mild drowsiness, mild dizziness and mild fever. However, AE relatedness to treatment with Taphalgin was estimated as unlikely. The AEs were self-limiting.

### Example 5.

Patient N, age 57 years, female.

Diagnosis: cervical cancer, pT2abNxMO, st. IIab. Chronic pain.

The patient cannot perform hard work but can perform light or sedentary work (such as household chores or office work) (70-80 on the Karnofsky scale).

Patient with uncontrolled pain (excluding breakthrough pain or pain from other causes not associated with persistent pain) despite treatment with weak opioid analgesics ± NSAIDs ± adjuvant treatments before treatment was administered according to the proposed administration pattern.

Pain was treated according to the proposed administration pattern using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 4-6 mg one to three times daily for 84 days. Pain intensity was evaluated at 1-1.5 hours before treatment, 1-1.5 hours, 4 hours and 12 hours after treatment.

During the first 5 days, the product was administered at a dose of 4 mg three times daily at intervals of 7-8 hours. Pain intensity was usually given an NRS score of 3-4 before dosing and 1-3 after dosing (at 1 hour and 4 hours). On treatment days 3-5, pain intensity score was 1-2 before treatment and 0 after treatment (at 1 hour and 4 hours). On days 6 to 42, the product was administered at 4 mg once daily. Pain intensity score was equal to 1-2 before dosing and declined to 0 after dosing (at 1 hour and 12 hours) up to treatment day 37. On days 37 to 42, pain intensity score was 2 before dosing, 1 at 1 hour after dosing and 1-2 at 4 hours after dosing. On days 43 to 84, the product was administered at a dose of 6 mg once daily. Pain intensity was usually given a score of 1 before dosing and 0 after dosing (at 1 hour and 12 hours after dosing).

No AEs associated with the analgesic drug Taphalgin were reported.

### Example 6.

Patient N, age 49 years, male.

Diagnosis: cancer of the right kidney, T4NOMI, St.IV. Moderate chronic pain.

The patient is undergoing treatment on an outpatient basis, he is capable of self-care but cannot perform work. He spends over 50% of waking time up and about (50-60 points on the Karnofsky scale).

Patient with uncontrolled pain (excluding breakthrough pain or pain from other causes not associated with persistent pain) despite treatment with weak opioid analgesics ± NSAIDs ± adjuvant treatments before treatment according to the proposed administration pattern.

The proposed administration pattern for pain control was employed using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 4 mg once daily for 83 days. Pain intensity was evaluated at 1-1.5 hours before treatment, 1-1.5 hours and 12 hours after treatment.

During the first two days of treatment with Taphalgin, pain intensity was usually given an NRS score of 3-4 before dosing and 0-2 after dosing (at 1 hour and 12 hours). From day three, pain intensity was usually given a score of 1 before dosing and 0 at 1 hour and 12 hours after dosing.

The overall physical and mental status of the patient and his sleep improved.

No Taphalgin-associated AEs were reported.

### Example 7.

Patient N, age 43 years, female.

Diagnosis: right breast cancer, T3NIMO, St. II B. Moderate chronic pain.

The patient cannot perform hard work but can perform light or sedentary work (such as household chores or office work) (70-80 on the Karnofsky scale).

Patient with uncontrolled pain (excluding breakthrough pain or pain from other causes not associated with persistent pain) despite treatment with weak opioid analgesics ± NSAIDs ± adjuvant treatments before treatment was administered according to the proposed administration pattern.

Pain was treated according to the proposed administration pattern using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 4 mg one to three times daily for 83 days. Pain intensity was evaluated at 1-1.5 hours before treatment, 1-1.5 hours, 4 hours and 12 hours after treatment.

During the first 3 days, NRS pain intensity was given a score of 1 before dosing and 2 at 1-4 hours after dosing. During these three days, the product was administered twice daily.

On subsequent days the product was administered at 4 mg once daily. Pain intensity score was usually 1 before dosing and 0 after dosing (at 1 hour and 12 hours after dosing). On isolated days, pain intensity score remained 0 throughout the day.

Reported AEs include mild numbness of the lower extremity, which was well tolerated by the patient, did not interfere with daily activities and caused minimum discomfort. Causal relationship with Taphalgin was evaluated as possible. The AEs was self-limiting and did not require treatment or Taphalgin dose adjustment.

### Example 8.

Patient N, age 67 years, female.

Diagnosis: left breast cancer ypT1NOMO, St. I. Moderate chronic pain.

The patient is undergoing treatment on an outpatient basis, is capable of self-care but cannot perform work. He spends over 50% of waking time up and about (50-60 points on the Karnofsky scale).

Patient with uncontrolled pain (excluding breakthrough pain or pain from other causes not associated with persistent pain) despite treatment with weak opioid analgesics ± NSAIDs ± adjuvant treatments before treatment was administered according to the proposed administration pattern.

Pain was treated according to the proposed administration pattern using Taphalgin solution for subcutaneous injection, a product on the basis of the tetrapeptide Tyrosyl-D-arginyl-phenylalanyl-glycinamide (H-Tyr-D-Arg-Phe-Gly-NH₂). Taphalgin was given by subcutaneous injection at an individualized single dose of 4 mg one to three times daily for 85 days. Pain intensity was evaluated at 1-1.5 hours before treatment, 1-1.5 hours, 4 hours and 12 hours after treatment.

During the first 5 days, the product was administered at a dose of 4 mg three times daily at intervals of 6-8 hours. During the first 3 days, NRS pain intensity was given a score of 3-4 before dosing and 0-1 at 1 hour and 4 hours after dosing. On days 4 and 5, pain intensity score was usually 2-3 before dosing and 0-1 at 1 hour and 4 hours after dosing.

On subsequent days the product was administered at a dose of 4 mg once daily. Pain intensity score was usually equal to 1 before dosing, declined to 0 after dosing and persisted at this level throughout the day.

The patient remarked improved physical and mental status.

No Taphalgin-associated AEs were reported.

### Example 9.

Patient N, age 43 years, female.

Diagnosis: uterine myoma

Surgical intervention: hysteroscopy, mini-laparotomy, myomectomy, sanitation of the pelvic cavity, active drainage of the abdominal cavity.

Taphalgin was administered at 2 mg three times daily to control early postoperative pain.

During the first day, NRS pain intensity score was initially equal to 6 before treatment, 6 at 30 minutes after dosing, 5 at 1 hour after dosing, 8 at 2 hours after dosing, 5 at 4 hours after dosing, and 3 at 6 hours after dosing. As the pain intensity score was 8, the patient was additionally given 500 mg of paracetamol. Subsequently, on days 1, 2 and 3 Taphalgin was the only product used for pain relief; NRS pain intensity score was 4-5 before dosing, declining to 3-2 at 0.5-1 hours after dosing and to 0-2 at 4-6 hours after dosing.

No AEs were observed.

### Example 10.

Patient N, age 30 years, female.

Diagnosis: infertility; bilateral hydrosalpinx.

Surgical intervention: laparoscopy, bilateral tubectomy, hysteroscopy, separate diagnostic curettage of the uterine cavity and cervical canal.

Taphalgin was administered at 4 mg twice daily to control early postoperative pain.

During the first day, NRS pain intensity score was initially equal to 4 before treatment with the product, 4 at 30 minutes after dosing, 3 at 1 hour after dosing, 3 at 2 hours after dosing, 6 at 4 hours after dosing, and 3 at 6 hours after dosing. As the pain intensity score was 6 (at 4 hours after the first dose of Taphalgin), the patient was additionally given 500 mg of paracetamol. Subsequently, on days 1 and 2, Taphalgin was the only product used for pain relief; NRS pain intensity score was 4-3 before dosing, declining to 3-2 at 1 hour after dosing and persisting at this level for 4-6 hours. On day 2, pain intensity score was 2 before dosing, declining to 0 after dosing and persisting at this level for 4-6 hours. No AEs were observed.

### Example 11.

Patient N, age 36 years, female.

Diagnosis: uterine myoma

Surgical intervention: mini-laparoscopy, hysteroscopy. Myomectomy without uterine cavity breach. Sanitation of the pelvic cavity.

Taphalgin was administered at 4 mg three times daily to control early postoperative pain.

During the first day, NRS pain intensity score was initially equal to 4 before treatment with the product, 4 at 30 minutes after dosing, 4 at 1 hour after dosing, 5 at 2 hours after dosing, 3 at 4 hours after dosing, and 2 at 6 hours after dosing. As the pain intensity score was 5 (at 2 hours after the first dose of Taphalgin), the patient was additionally given 500 mg of paracetamol. Subsequently, on days 1, 2 and 3 Taphalgin was the only product used for pain relief; NRS pain intensity score was 2-1 before dosing, and 1 for 6 hours after dosing.

An AE in the form of elevated ESR was observed. The AE was mild, did not require specialized treatment or adjusting the dose of the drug, and resolved in recovery without sequelae. Causal relationship with Taphalgin was evaluated as possible.

### Example 12.

Patient N, age 31 years, male.

Diagnosis: inguinal hernia on the right.

Surgical intervention: laparoscopic inguinal hernia repair with an implant on the right.

Taphalgin was administered at a dose of 4 mg three times daily for treating early postoperative pain.

During the first day, NRS pain intensity score was initially equal to 6 before treatment, 4 at 30 minutes after dosing, 2 at 1 hour after dosing, 1 at 2 hours after dosing, 0 at 4 hours after dosing, and 1 at 6 hours after dosing. Subsequently, on days 1 and 2, NRS pain intensity score was 2-1 before dosing and 1-0 for 6 hours after dosing. On day 3, pain intensity score was 1 before dosing, declining to 0 after dosing and persisting at this level for 6 hours.

No AEs were observed.

### Example 13.

Patient N, age 51 years, female.

Diagnosis: urinary incontinence.

Surgical intervention: cystoscopy. Mid-urethral sling urethropexy with a Urosling synthetic sling.

Taphalgin was administered at 2 mg three times daily for treating early postoperative pain.

During the first day, NRS pain intensity score was initially equal to 3 before treatment, 2 at 30 minutes after dosing, 2 at 1 hour after dosing, 2 at 2 hours after dosing, 2 at 4 hours after dosing, and 3 at 6 hours after dosing. Subsequently on days 1, 2 and 3, NRS pain intensity score was 2-1 before dosing and 1-0 for 6 hours after dosing.

No AEs were observed.

### Example 14.

Patient N, age 37 years, female.

Diagnosis: asymptomatic cyst (dermoid) of the right ovary. Laboratory tests and magnetic resonance imaging ruled out malignancies.

Surgical intervention: laparoscopy, adhesiolysis, ovariectomy on the right.

Taphalgin was administered at 2 mg three times daily to control early postoperative pain.

During the first day, NRS pain intensity score was initially equal to 4 before treatment, 3 at 30 minutes after dosing, 1 at 1 hour after dosing, 1 at 2 hours after dosing, 0 at 4 hours after dosing, and 0 at 6 hours after dosing. Subsequently, on days 1 and 2, NRS pain intensity score was 1 before dosing and 0 for 6 hours after dosing.

No AEs were observed.

To draw an overall conclusion, we note that these studies provide evidence of the efficacy and safety of the claimed medicinal product for use in administering in the treatment of pain and the great potential that its application holds in the early postoperative period after a variety of surgical interventions and in long-term pain treatment in cancer patients.

Some of the most important features of biological activity of the tetrapeptide H-Tyr-D-Arg-Phe-Gly-NH2 and Taphalgin, a drug on the basis thereof, include being free from serious side effects, such as CNS disturbances manifesting in impaired consciousness and euphoria or effects on the respiratory centre. Another positive feature is the wide therapeutic range free of significant side effects. These features justify possible uses of the claimed methods for the control, treatment and prevention of pain in much more extensive areas of therapy, including uses outside the hospital, such as under field or household conditions, by health practitioners of modest qualifications or by the patients themselves, which is extremely important for the treatment of cancer pain and for the treatment of early postoperative pain after a variety of surgical interventions.

## Claims

1. A medicinal product for use in the treatment, control and/or prevention of pain, wherein an administration pattern of the medicinal product includes administering tetrapeptide Tyrosyl-D-arginyl-phenylalanyl-glycinamide (H-Tyr-D-Arg-Phe-Gly-NH₂) at a single dose of 2 mg to 7 mg, for a daily dose not exceeding 42 mg, with a dosing interval of 4 hours or more.

2. The medicinal product for use according to claim 1, wherein the administration pattern includes administering the tetrapeptide for pain relief in cancer patients or in the early postoperative period after a variety of surgical interventions.

3. The medicinal product for use according to claims 1-2, wherein the administration pattern includes administering the tetrapeptide Tyrosyl-D-arginyl-phenylalanyl-glycinamide (H-Tyr-D-Arg-Phe-Gly-NH₂) in the form of a solution for subcutaneous injection.

4. The medicinal product for use according to claims 1-3, wherein the administration pattern includes administering the tetrapeptide while monitoring the severity of pain and/or the patient's response to the tetrapeptide, and increasing or decreasing as necessary the single dose and/or number of doses per day and/or the dosing interval and/or the duration of the course of treatment.

5. The medicinal product for use according to claims 1-4, provided that the medicinal product is for use in administering in the treatment of pain, wherein, the administration pattern includes performing, depending on the severity of pain, tetrapeptide injections daily or at intervals of one to several days.

6. The medicinal product for use according to claims 1-5, provided that the medicinal product is for use in administering in the treatment of pain, wherein the administration pattern includes a duration of the pain treatment course of up to 96 days, with the possibility of repeating the course multiple times.

7. The medicinal product for use according to claims 1-6, provided that the medicinal product is for use in administering in the treatment of pain, wherein the administration pattern includes determining the total duration of pain treatment by the patient's condition; wherein in cancer patients the duration of a single course of pain treatment is preferably up to 96 days or more, with the possibility of repeating the course multiple times; wherein repeating the course is recommended after a pause of at least 2-5 days between the courses, with the number of repeat courses being determined by the patient's condition.

8. The medicinal product for use according to claims 1-7, provided that the medicinal product is for use in administering in the treatment of pain, wherein the administration pattern includes determining the total duration of treatment by the patient's condition; wherein in patients with pain in the early postoperative period after a variety of surgical interventions, the duration of pain treatment is 1 to 10 days.

9. The medicinal product for use according to claims 1-8, wherein the administration pattern includes using a single 4 mg to 7 mg dose of the tetrapeptide.

10. The medicinal product for use according to claims 1-9, wherein the administration pattern includes a recommended starting single dose of the tetrapeptide Tyrosyl-D-arginyl-phenylalanyl-glycinamide (H-Tyr-D-Arg-Phe-Gly-NH₂) which is 4 mg.

11. The medicinal product for use according to claims 1-10, wherein the administration pattern includes increasing or reducing as necessary the single dose in line with an increase or reduction of the dose by 25-50% relative to the previous value.

12. The medicinal product for use according to claims 1-11, wherein the administration pattern includes administering the tetrapeptide at a dose of 4 mg 2-3 times daily.

13. The medicinal product for use according to claims 1-4 and 9 to 12, provided that the medicinal product is for use in administering to a human in the prevention of pain, wherein the administration pattern includes administering the tetrapeptide when there is a risk of an attack of pain in cancer patients or in the early postoperative period after a variety of surgical interventions.

14. A medicinal product for use according to any of the claims 1-13, which comprises the tetrapeptide Tyrosyl-D-arginyl-phenylalanyl-glycinamide (H-Tyr-D-Arg-Phe-Gly-NH₂) and excipients: sodium chloride, mannitol, glycine, sodium acetate trihydrate, acetic acid and water at the following ratio of the components, wt %:
| | |
|---|---|
| Tetrapeptide | 0.380 - 0.420 |
| Sodium chloride | 0.475 - 0.525 |
| Mannitol | 0.475 - 0.525 |
| Glycine | 0.475 - 0.525 |
| Sodium acetate trihydrate | 0.0380 - 0.0420 |
| Acetic acid | up to pH 4.0 - 6.0 |
| Water | up to 100 |

15. The medicinal product for use according to claim 14 that is **characterized by** the following ratio of the components, wt %:
| | |
|---|---|
| Tetrapeptide | 0.40 |
| Sodium chloride | 0.50 |
| Mannitol | 0.50 |
| Glycine | 0.50 |
| Sodium acetate trihydrate | 0.04 |
| Acetic acid | up to pH 4.0 - 6.0 |
| Water | up to 100 |

16. The medicinal product for use according to claim 14 that is **characterized by** the following ratio of the components, mg:
| | |
|---|---|
| Tetrapeptide | 3.8 - 4.2 |
| Sodium chloride | 4.75 - 5.25 |
| Mannitol | 4.75 - 5.25 |
| Glycine | 4.75 - 5.25 |
| Sodium acetate trihydrate | 0.38 - 0.42 |
| Acetic acid | up to pH 4.0 - 6.0 |
| Water | up to 1 mL |

17. The medicinal product for use according to claim 16 that is **characterized by** the following ratio of the components, mg:
| | |
|---|---|
| Tetrapeptide | 4.00 |
| Sodium chloride | 5.00 |
| Mannitol | 5.00 |
| Glycine | 5.00 |
| Sodium acetate trihydrate | 0.40 |
| Acetic acid | up to pH 4.0 - 6.0 |
| Water | up to 1 mL |

18. The medicinal product for use according to claims 14-17, which is or is prepared in the dosage form of a solution for subcutaneous injection.
